Europäisches Patentamt

(19) European Patent Office

Office européen des brevets

(11) Publication number: **0 151 855**
**B1**

(12) **EUROPEAN PATENT SPECIFICATION**

(45) Date of publication of patent specification: **11.05.88**

(51) Int. Cl.⁴: **C 12 M 1/34**

(21) Application number: **84306067.4**

(22) Date of filing: **05.09.84**

(54) Identification of microorganisms by infrared analysis of evolved carbon dioxide.

(30) Priority: **05.12.83 US 557985**

(43) Date of publication of application:
**21.08.85 Bulletin 85/34**

(45) Publication of the grant of the patent:
**11.05.88 Bulletin 88/19**

(84) Designated Contracting States:
**BE DE FR GB IT NL SE**

(56) References cited:
**EP-A-0 104 463**
**FR-A-2 256 246**

**JOURNAL OF FOOD SCIENCE, vol. 47, no. 4,
July/August 1982, pages 1222-1225, US; C.H.
THRELKELD: "Detection of microbial
contamination utilizing an infrared CO2
analyzer"**

(73) Proprietor: **Becton, Dickinson and Company
Mack Centre Drive P.O. Box 2224
Paramus New Jersey 07652-1149 (US)**

(72) Inventor: **Perks, H. Mark
935 Litchfield Road
Baltimore Maryland (US)**
Inventor: **Ahnell, Joseph E.
4519 Hydes Road
Hydes, Baltimore Maryland (US)**
Inventor: **McCarthy, Laurence R.
7 Stratford Road
Baltimore Maryland (US)**

(74) Representative: **Ruffles, Graham Keith et al
MARKS & CLERK 57-60 Lincoln's Inn Fields
London WC2A 3LS (GB)**

## Description

### Field of the Invention

The present invention relates generally to the identification of microorganisms. More particularly, the present invention relates to the identification of an unknown microorganism by comparison of the carbon dioxide evolution profile of the unknown microorganism generated from growth in a plurality of substrates with profiles of known microorganisms generated from growth in the same plurality of substrates.

### Description of the Prior Art

Conventional methods of organism identification usually involve culturing techniques. In general, this procedure consists of inoculating a sample in a suitable growth medium and growing any microorganisms present in the sample to a concentration such as to allow plating of the microorganism onto a solid agar medium. The microorganism is subsequently recovered as a relatively pure concentrated sample. The microorganism is then identified as to colonial morphology and Gram reaction by observation under a microscope. Culture techniques are time consuming and are often not completely definitive. There is a need for a more rapid and definitive means for microorganism identification.

The generation and use of profiles of standard microorganisms generated from a plurality of substrates for the identification of unknown microorganisms is known. A commercial system for identification of bacteria from clinical samples is available under the trademark SCEPTOR from BBL Microbiology Systems division of Becton, Dickinson and Company. In the use of this system, an unknown specimen is incubated in a plurality of individual growth substrates in separate wells of a panel. Metabolic activities such as oxidation of carbohydrates or deamination or decarboxylation of amino acids result in color changes in those wells in which the microorganism utilizes the substrate in that well. Identification is accomplished by comparing a profile of the metabolic activities of the unknown microorganism with profiles of various known microorganisms.

Another system for identification of microorganisms which utilizes the metabolism of Carbon-14 labeled substrates to produce radiolabeled carbon dioxide is disclosed in United States Patents 3,946,496 and 4,057,470 to Schrot. In the Schrot method, the evolved $^{14}CO_2$ is collected and assayed by nuclear counting means in order to construct radiorespirometric profiles. The method disclosed in the Schrot patents requires expensive radiolabeled substrates and nuclear counting equipment. In addition, it is necessary to count the $^{14}CO_2$ evolved from each substrate for a period of time sufficient to develop detection and rate data.

Infrared detection of $CO_2$ in head space gas as a means of determining microbial contamination in commercial food products has been described by C. H. Threlkeld (J. of Food Science, 47, 1225 (1982). This method, however, deals only with detection and is not concerned with identification.

It is thus the principal object of the present invention to provide a rapid means for the identification of microorganisms. Another object of the invention is to provide a means for rapid organism identification which does not depend upon radio-labeled substrates. It is an additional object of the invention to provide identification system for microorganisms which does not depend upon chemical indicators or other added indicating reagents to classify reaction results. It is a further object of the invention to provide a process amenable to automated detection means so as to eliminate inconsistencies present when reaction results are interpreted by the human eye.

### Summary of the Invention

The present invention provides a method for identification of microorganisms in a sample, such as blood, urine, spinal fluid, water and the like. The invention comprises inoculating a plurality of substrates with a sample of the unknown microorganism. The substrates include various different carbon sources, such as carbohydrates and amino acids, as well as other components required for growth of the microorganism. Each of the substrates may be metabolized by certain specific microorganisms. The inoculated substrates are incubated for a predetermined length of time sufficient to permit metabolic breakdown of some of the carbon source to produce carbon dioxide. The occurrence of metabolism of each carbon source by the microorganism to carbon dioxide is determined by infrared analysis of the gas produced by metabolism of any microorganism present in the sample. The detection of carbon dioxide is taken as a positive indication of metabolism of the carbon source. Similarly, the failure of the unknown organism to produce carbon dioxide in amounts detectable by infrared analysis is taken as a negative indication of metabolism of that specific carbon source. A profile of the unknown organism is obtained by noting the positive and negative metabolic results obtained using a plurality of carbon sources. The profile thus obtained is compared to profiles obtained from known microorganisms. The unknown microorganism is identified by the correspondence of its profile with that of one of the known microorganisms.

It should be understood that the profiles generated for known microorganisms by the method of this invention may differ from profiles generated by other methods for the same organisms. Other methods utilize acid-base indicators present in the sample to indicate positive reactions by color change. In these methods, fermentation reactions are detected by acid production and subsequent drop in pH to indicate positivity, while decarboxylation reactions are detected by a rise in pH. Carbon dioxide may or may not be prdouced as a consequence of these or similar metabolic reactions.

The process of the invention may be conducted in an apparatus which comprises a container for a plurality of different and separate carbon sources, means for inoculating each of the containers with a sample of the unknown microorganism, means for exposing the inoculated containers to conditions conducive to the occurance of normal metabolic processes, and infrared means for analyzing carbon dioxide gas produced as a result of metabolism of the carbon sources to determine whether or not metabolism occurred in each specific growth medium.

A Brief Description of the Drawings

Fig. 1 shows an apparatus suitable for use in the present method.

Fig. 2 shows the infrared absorption spectrum of carbon dioxide and the absorption band between 2300 and 2400 cm$^{-1}$.

Figs. 3, 4 and 5 show the infrared absorption spectrum of carbon dioxide in the area of interest for polymethylpentene, borosilicate glass and soda lime glass containers respectively.

Figs. 6 and 7 represent the infrared scan of polymethylpentene plastic and borosilicate glass containers which contain various levels of carbon dioxide.

Figs. 8 and 9 show carbon dioxide production from the metabolism of microorganisms in various growth media.

Figs. 10 and 11 show positive and negative carbon dioxide production by microorganism metabolism of various carbon sources.

Detailed Description of the Invention

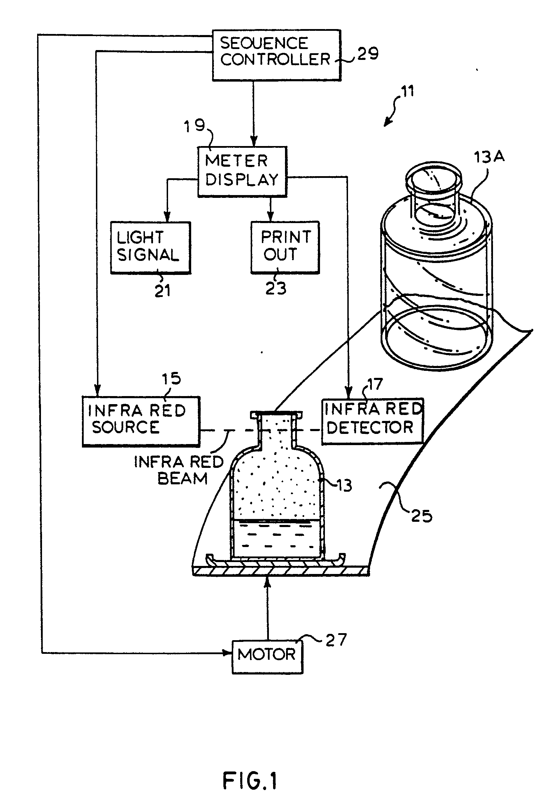

An apparatus suitable for the practice of the invention is designated in Figure 1 by the numeral 11. In apparatus 11 useful in the present invention, the presence or absence of $CO_2$ produced by metabolism of a microorganism in a growth medium is detected by passing an infrared beam through the sidewall of a container and detecting the infrared absorption of the gas produced by metabolism within the container. The container may be a vial or any other single or multi-channel container.

A sample to be analyzed, such as a patient specimen, for example, blood, urine, or the like, or an organism grown in culture from a variety of sources (water, soil, food stuffs, patient specimens, etc.), is placed into a sterile container, 13, together with a growth medium suitable to produce $CO_2$ upon metabolism of a microorganism. Thereafter the sample is incubated in the container. At suitable intervals the container is transferred to an infrared measuring means which includes an infrared source 15 and an infrared detector 17. The infrared absorption within the head space of the container is detected and may be displayed on a meter 19 which may include provision for simultaneous display of a light signal 21 and a computer printout 23.

The container 13 is located on a track 25, which may be linear, circular, or of serpentine geometry to provide easy positioning of the container 13 between the infrared source and the infrared detector. A second culture container 13A is shown in phantom outline in position waiting to be tested by infrared source 15 and infrared detector 17. A motor 27 is provided to activate the track 25. A sequence controller 29 is used to properly sequence activation of the motor 27, the infrared source 15 and the meter display 19. It may sometimes be desirable to provide means for rotating a cylindrical container during activation of the infrared source to provide compensation for uneven sidewall thickness and additionally to provide means for aligning the containers in the center of the infrared beam.

The culture containers 13 and 13A may be glass or plastic containers, such as vials, fitted with rubber septa and sealed by means of aluminum closures, or may be separate but contiguous wells of a molded plastic cartridge or tray or linear geometry. Other materials and geometry satisfactory for the performance of the required gas analysis may be employed if desired. The containers will have a total capacity of between about 1 ml and about 200 ml, preferably between about 30 ml and about 150 ml, of which preferably 2—100 ml will be occupied by the culture medium and test sample. The volume of blood or urine or other sample may be, for example, 0.1—10 ml.

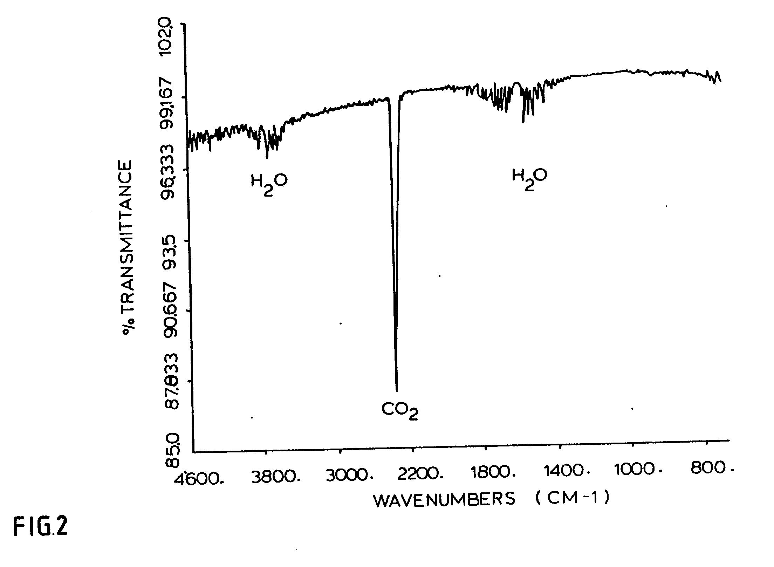

The container must have a "window" providing at least about one percent transmittance at the band width suitable for detecting the gaseous product from metabolism of the growth medium. Carbon dioxide has a strong infrared absorption band at 2349 cm$^{-1}$, shown in Figure 2, free from water vapor interference. The required transparent window of the container when using this absorption band thus includes wave numbers from about 2300 cm$^{-1}$ to about 2400 cm$^{-1}$. Carbon dioxide also has a low frequency absorption band at 670 cm$^{-1}$ which is of use in the practice of this invention.

It has been discovered that certain types of glass containers and a particular type of plastic container are useful in the practice of the present invention. In particular, borosilicate glass containers and soda lime glass containers have been found to be useful. It has also been determined that polymethylpentene plastic provides a transparent "window" at the absorption wavelength of carbon dioxide. Other glass or plastic materials may also be used.

It should be understood that those skilled in the art of infrared spectroscopy do not consider either glass or plastic to be of use as a sample-containing cell for infrared spectroscopy in the usual infrared absorption range of 400 cm$^{-1}$ to 4000 cm$^{-1}$. Glass, although transparent to visible light, becomes opaque to infrared wavelengths just slightly longer than those applicable to carbon dioxide analysis. Plastics, being organic materials, have infrared absorption bands throughout the usual range. It is surprising that polymethylpentene exhibits transparent "windows" at 2349 cm$^{-1}$ and near 600 cm$^{-1}$ with enough transmission to permit the analysis of carbon dioxide at either of the above absorption frequencies.

While not wishing to be bound by any theory, it appears that carbon dioxide absorbs infrared energy in a wave length region corresponding to the covalent triple bond of organic molecules. Triple bonds are not normally found in polymeric materials. Polymethylpentene is a preferred polymeric material containing covalent double bonds. For use as a container in the method of the invention, the polymeric material must also be capable of being sterilized, whether by conventional autoclave or gas or radiation sterilization techniques. Polymethylpentene is suitable in this respect in that it can withstand temperatures suitable to achieve sterility.

The growth medium which is to be inoculated with the microorganism may be composed of the single carbon source and a basal medium containing salts, buffers, growth factor(s) and the like. Alternatively, in instance where the buffering capacity and ionic strength of the medium is not of consequence, and where growth factor enhancement is not required, one or more of these components may be left out of the medium. Exemplary of suitable basal media which may be used for the practice of this invention are Carbon Assimilation Medium, hereinafter referred to as CAM, SCEPTOR Gram-negative MIC—ID dehydrated culture medium, hereinafter referred to as DCM, *E. Coli* basal medium 1, and Ledbetter medium. Such standard media are described in the *Manual of Clinical Microbiology,* Third Edition, American Society for Microbiology, 1980, edited by E. H. Lennette, E. H. Spaulding and J. P. Truant; in *Experiments in Molecular Genetics,* 1968 and 1972, edited by R. C. Cloroes and W. Hayes, Blacknell Scientific Publications; and in P. E. Goldenbaum and G. A. Hall, *J. Bacterial. 140,* p. 459.

The basal media may be prepared and the required carbon sources added prior to being dispensed into the container, or the carbon sources may be present in the containers or cartridge wells in liquid, gel, frozen, elution disk or other dehydrated form and the basal medium added thereto.

Among the carbon sources which may be used are carbohydrates, such as glucose, lactose, arabinose, raffinose, sucrose, rhamnose, trehalose and the like; carbohydrate derivatives such as salicin; acid sugars; urea; polyols such as glycerol, mannitol, inositol, sorbitol, dulcitol, adonitol and the like; Krebs cycle intermediate acids such as citric acid; amino acids such as arginine, glycine, alanine, lysine, ornithine, tyrosine, threonine, histidine, leucine and the like; low molecular weight peptides; purine and pyrimidine bases; and low molecular weight compounds capable of being decarboxylated to produce carbon dioxide, such as fatty acids. The carbon source should be present in sufficient quantity to provide a readily detectable amount of carbon dioxide upon metabolism. Preferably, the growth medium contains from about two percent to about ten percent, on a weight basis, of the carbon source.

The growth medium containing the carbon source in the container is inoculated with a small amount of a sample containing the microorganism. Inoculation may be performed either manually or by automated means to prepare either a multiplicity of containers or multiple compartments within a common vessel.

After the inoculation step, the microorganisms are incubated at a temperature of between about 20°C and about 45°C, for a period of from about 1 to about 24 hours depending on the microorganism and the carbon source, but preferably for from about 2 to about 8 hours. Some organisms achieve optimum growth at temperatures of 20°C or lower while others may exhibit optimum growth at 45°C or higher. Any temperature and duration of incubation best suited in a given circumstance may be employed. Although satisfactory growth can be achieved without agitation, metabolism preferably is carried out with active shaking, stirring, or the like, effective to insure proper evolution of $CO_2$ from the medium. In one preferred embodiment, agitation is provided by stirring or rotary shaking to introduce a vortex in the liquid medium.

Determination of the presence of $CO_2$ is effected by infrared analysis of the gas evolved from the culture medium. The absorption of infrared radiation at about 2360 $cm^{-1}$ is used for this analysis. This absorption by $CO_2$ is shown in the infrared absorption spectrum of $CO_2$ in Figure 2.

In the practice of this invention, infrared absorption is determined immediately after inoculation and periodically thereafter up to the conclusion of the incubation period. Total absorption at about 2360 $cm^{-1}$ is measured. Corrections for absorption by the containers are then made by measuring absorption of glass containers at 2400 $cm^{-1}$ and of polymethylpentene containers at 2250 $cm^{-1}$. Subtraction of the reference absorptions of the containers from the total absorptions measured provides the corrected absorption due to the evolved $CO_2$.

A profile of the utilization or non-utilization (as determined by the presence or absence of carbon dioxide in the head space gas) of the carbon sources is used to generate a profile of the unknown microorganism in the sample. A series of profiles of known microorganisms are similarly prepared. Comparison of the profile of the unknown with the known profiles permits identification of the unknown. It is evident that this comparison is facilitated by first separating the profiles of the unknown and the known organisms into groups according to standard known classifications, such as the Gram stain, i.e., gram positive or gram negative.

The following examples further illustrate various features of the invention but are intended to in no way limit the scope of the invention.

## Example 1

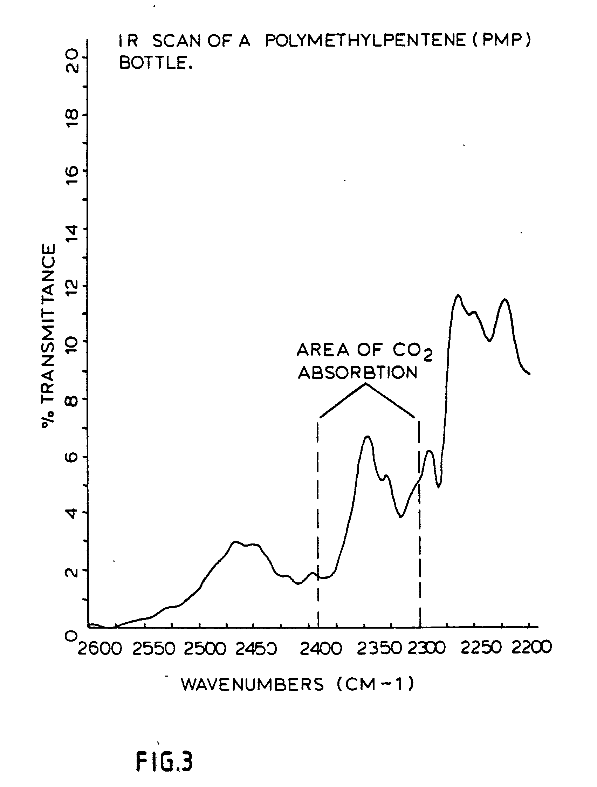

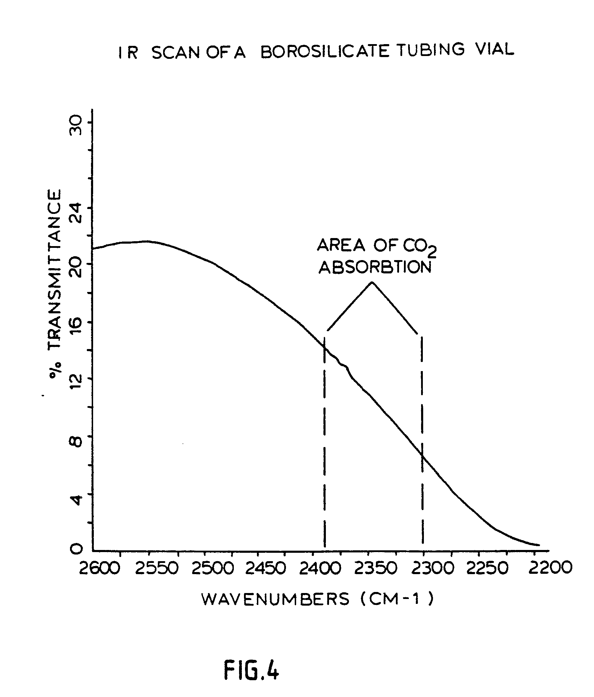

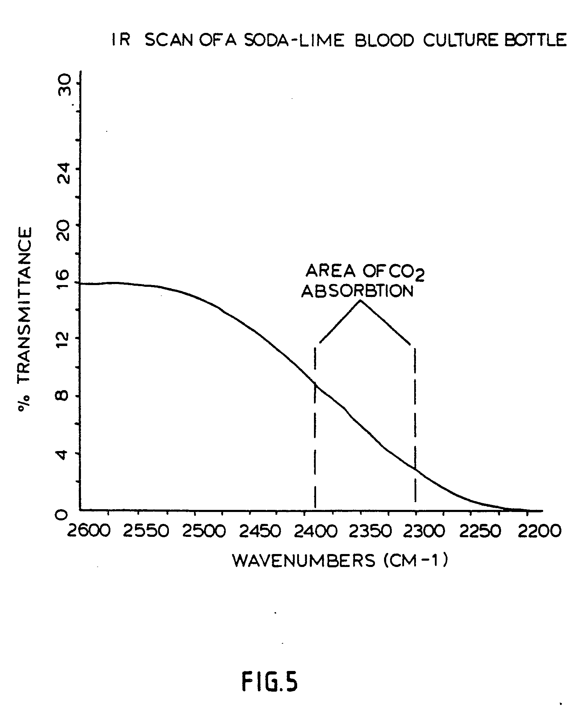

The relative amounts of infrared transmission through three different materials is shown in Figures 3 to 5. In Figure 3, a 125 ml polymethylpentene bottle with the sidewall thickness of approximately .03 inches and an outside diameter of 1.88 inches is subjected to an infrared scan. The area of $CO_2$ absorption is outlined in Figure 3. As indicated, the area of $CO_2$ absorption varies from a transmittance of from about 2 percent to about 7 percent. This is sufficient to permit a scan for $CO_2$ to be made. In Figure 4, a tubing vial of borosilicate glass with a sidewall thickness of 0.053 inches and an outside diameter of 1.33 inches is scanned at the wave numbers of interest. It is seen that the transmittance of borosilicate glass in the infrared wave number region slightly beyond the area of $CO_2$ absorption is zero. The percent transmittance in the area of $CO_2$ absorption varies from about 7 percent to about 14 percent. In Figure 5, a fifty milliliter bottle made of soda lime glass having an outside diameter of approximately 1.7 inches is scanned by infrared. Again, the percent transmittance at infrared wave numbers slightly greater than that for $CO_2$ absorption is zero. In the area of $CO_2$ absorption the percent transmittance ranges from about 3 to about 9. All infrared scans in this example and other examples were made with a Nicolet 5—MX FT—IR spectrophotometer. The scans for Figures 3 through 5 were made with the vials opened to room air and are the result of the addition and averaging of sixty scans. The scans are referenced against the scan of room air, which was also scanned sixty times. In each case the area of interest in terms of carbon dioxide absorption is in the region between approximately 2400 to 2200 cm$^{-1}$.

## Example 2

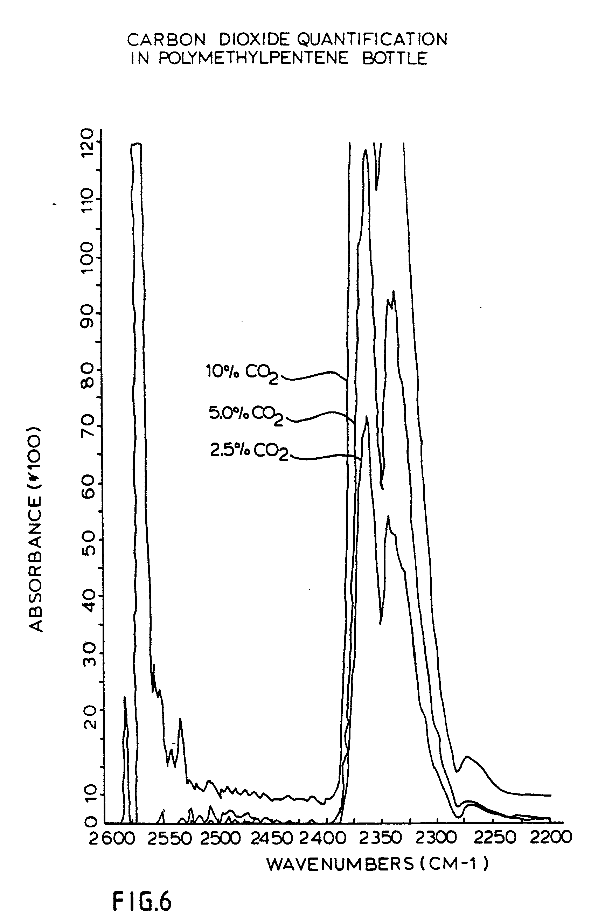

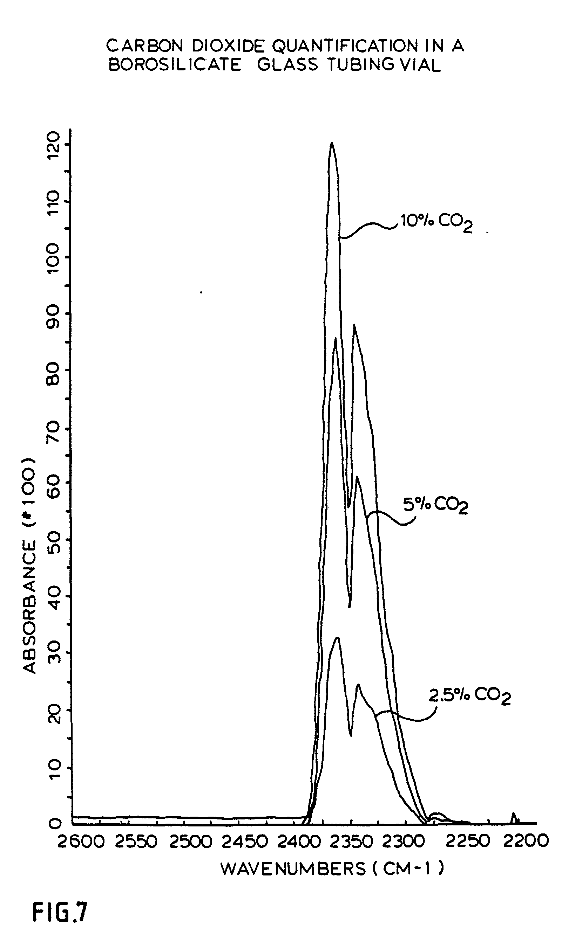

In Figures 6 and 7, various levels of carbon dioxide gas were provided in the head space of the polymethylpentene bottle and a borosilicate glass tubing vial respectively. Reference scans of each container were first taken with the bottles opened to room air by taking sixty readings in the Nicolet spectrophotometer. The vials were then flushed with gases containing approximately 2.5 percent, 5.0 percent and 10.0 percent carbon dioxide and were then quickly sealed. The bottle and the vial were then again read following each flushing for sixty scans in the Nicolet spectrophotometer. The results are shown in Figures 6 and 7 with the vertical axis in this case being the infrared absorbance times 100. A clear correlation can be seen between the increase in infrared absorption in the area between 2400 cm$^{-1}$ and 2300 cm$^{-1}$ with the increase in carbon dioxide concentration within the vials.

## Example 3

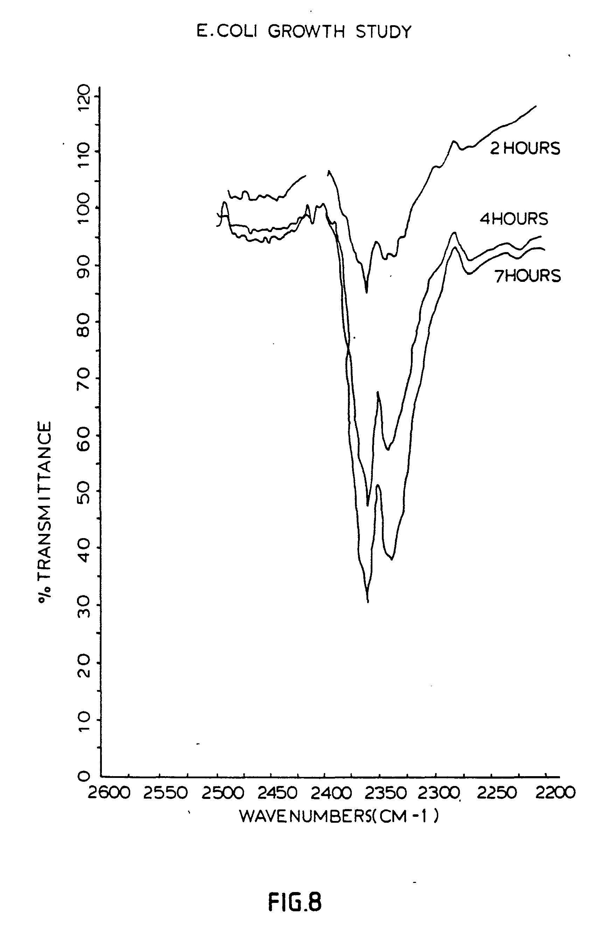

The increase in infrared absorption as correlated with organism growth is shown in Figure 8.

30 Mls of tryptic soy broth (TSB) contained in two polymethylenepentene bottles was sterilized by autoclaving. One bottle was inoculated at 0 hours with approximately one-half ml of a culture which had been grown overnight. The other bottle was not inoculated and was used as a reference. The bottles were scanned sixty times each at the intervals indicated in Figure 8. The data presented in Figure 8 represent the scan of the inoculated bottle minus the scan of the uninoculated bottle. The bottles were incubated at 37°C between readings. A clear correlation can be seen between organism growth and infrared absorption in the area of carbon dioxide absorption.

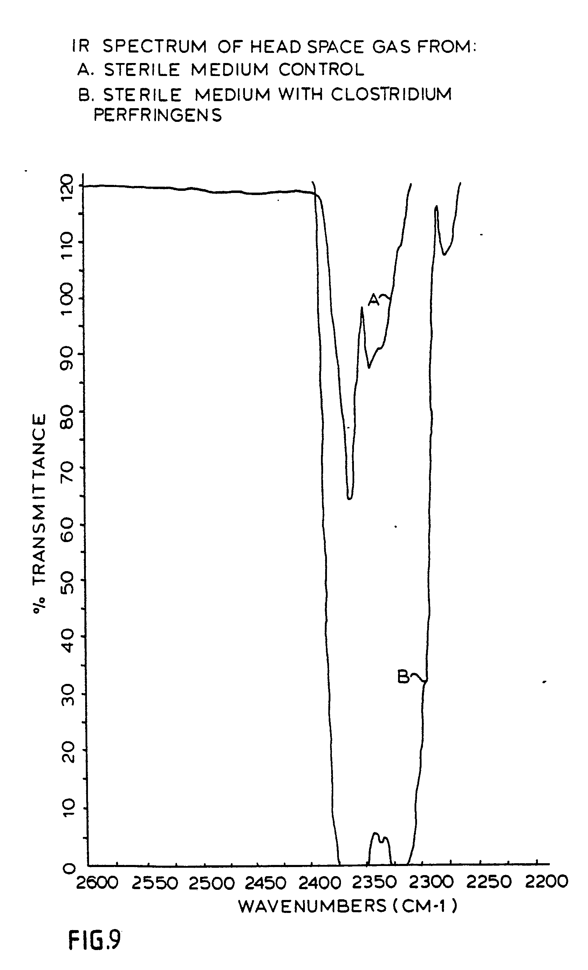

Figure 9 shows a scan of a soda lime glass vial containing sterile medium and a scan of a soda lime vial containing sterile medium inoculated with *Clostridium perfringens.* Both infrared scans were referenced against a scan of a soda lime glass vial open to room air. All scans were performed sixty times in the Nicolet spectrophotomer. The vial containing *C. perfringens* exhibits a much larger carbon dioxide signal than the vial containing the culture medium alone.

## Example 4

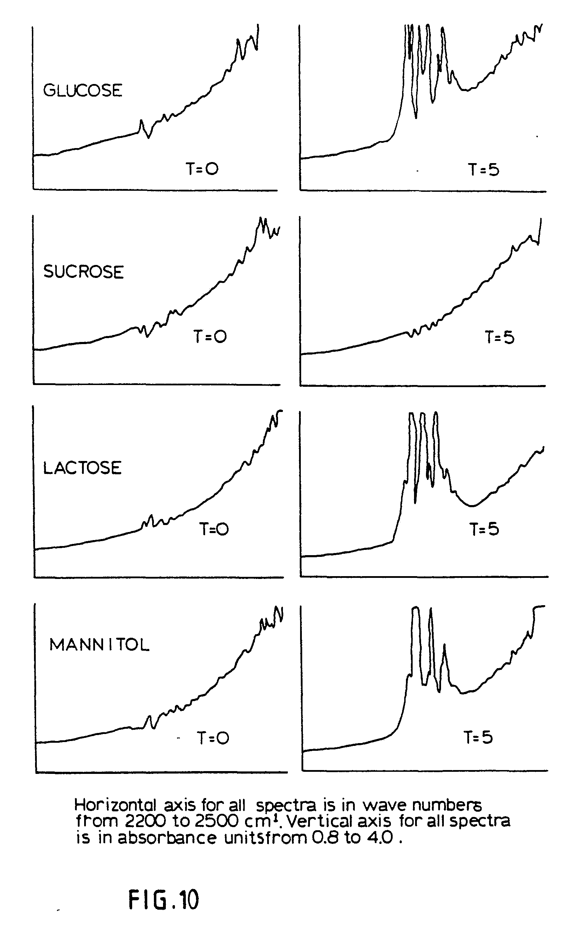

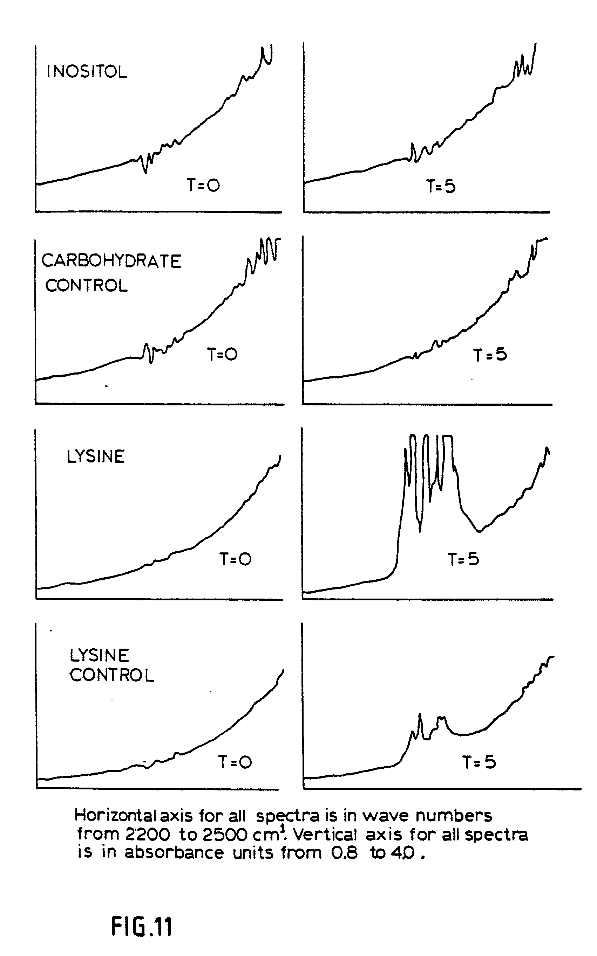

Vials of soda lime glass containing 30 ml of either CAM or DCM and selected carbon sources were inoculated with 5 ml of a suspension of approximately $10^9$ cells per ml of E. coli in normal saline. The infrared absorption was measured at time zero. Incubation was maintained at 37°C and the infrared absorption was measured after 5 hours. The results of this experiment are shown in Figures 10 and 11. Carbon dioxide was evolved and detected in the evolved gas as a result of metabolism by the microorganism of glucose, lactose, mannitol and lysine. The organism did not metabolize sucrose or inositol.

While the method and apparatus of the present invention have been described with respect to the detection of bacteria, the method and apparatus may be used to detect biological activity broadly, including cell organisms, tissue culture cells, yeasts, enzymatic reactions and the like. Also, this technology may be applied to the assessment of the susceptibility of a microorganism to an antimicrobial agent, wherein the production of carbon dioxide by the organism may either increase or decrease in response to the effect of the agent on the organism's growth.

## Claims

1. A method for identification of a microorganism in a sample, the method comprising:

(i) providing a plurality of containers wherein individual containers are each provided with a different growth medium for microorganisms, each growth medium including a respective carbon source which might be metabolizable to produce carbon dioxide;

(ii) preparing a standard carbon dioxide profile for a particular known microorganism by the following steps:

(a) inoculating the plurality of containers with a sample of the known microorganism,

(b) incubating the container for a time sufficient to cause metabolic breakdown of the growth media thereby resulting in the production of carbon dioxide from at least some of the growth media,

(c) detecting the presence or absence of carbon dioxide in the gaseous atmosphere evolved from each growth medium by measuring the infrared absorption of the gaseous atmosphere by passing an infrared beam through the gaseous atmosphere and detecting the infrared absorption of the gaseous atmosphere,

(d) preparing a standard carbon dioxide profile for the known microorganism based on the utilization of growth media wherein the presence or absence of carbon dioxide is determined by infrared absorption;

(iii) repeating steps (a) through (d) for a plurality of known microorganisms to provide a series of standard carbon dioxide profiles for known microorganisms;

(iv) repeating steps (a) through (d) for a sample containing an unknown microorganism to provide an unknown carbon dioxide profile; and

(v) comparing the unknown carbon dioxide profile with the series of standard carbon dioxide profiles with a view to identifying the unknown microorganism.

2. A method in accordance with Claim 1 wherein the infrared absorption is measured in glass containers at about 2360 cm$^{-1}$ and at about 2400 cm$^{-1}$, and the absorption at about 2400 cm$^{-1}$ is subtracted from the absorption at about 2360 cm$^{-1}$ thereby to provide a corrected measurement of the absorption at about 2360 cm$^{-1}$ due to carbon dioxide.

3. A method in accordance with Claim 1 wherein the infrared absorption is measured in polymethylpentene containers at about 2360 cm$^{-1}$ and at about 2250 cm$^{-1}$, and the absorption at about 2250 cm$^{-1}$ is subtracted from the absorption at about 2360 cm$^{-1}$ to provide a corrected measurement of the absorption at about 2360 cm$^{-1}$ due to carbon dioxide.

4. A method in accordance with Claim 1 wherein the infrared absorbance is measured at about 670 cm$^{-1}$.

5. A method in accordance with any of Claims 1 to 4 wherein the incubation is for 1 to 24 hours.

6. A method in accordance with Claim 5 wherein the incubation is for 2 to 8 hours.

7. A method in accordance with any of Claims 1 to 6 wherein the carbon source is selected from a carbohydrate, a carbohydrate derivative, a polyol, urea, citric acid, a fatty acid, an amido acid, a low molecular weight peptide, or a purine or pyrimidine base.

8. A method in accordance with Claim 7 wherein the carbon source is a carbohydrate.

9. A method in accordance with Claim 8 wherein the carbohydrate is selected from glucose, lactose, arabinose, raffinose, sucrose, rhamnose, or trehalose.

10. A method in accordance with Claim 7 wherein the carbon source is an amino acid.

11. A method in accordance with Claim 10 wherein said amino acid is selected from arginine, ornithine, lysine, glycine, alanine, tyrosine, threonine, histidine, or leucine.

12. A method in accordance with Claim 7 wherein the carbohydrate derivative is a salicin or the polyol is selected from glycerol, mannitol, inositol, sorbitol, dulcitol, or adonitol.

## Patentansprüche

1. Verfahren zur Identifizierung eines Mikroorganismus in einer Probe, bei dem

(i) eine Vielzahl von Behältern verwendet wird, von denen jeder mit einem verschiedenen Nährmedium für Mikroorganismen versehen ist, wobei jedes Nährmedium eine entsprechende Kohlenstoffquelle enthält, die unter Bildung von Kohlendioxid metabolisierbar sein könnte;

(ii) ein Standard-Kohlendioxid-Profil für einen bestimmten, bekannten Mikroorganismus mittels folgender Stufen erstellt wird:

(a) die Vielzahl von Behältern wird mit einer Probe des bekannten Mikroorganismus beimpft,

(b) die Behälter werden ausreichend lange inkubiert, um einen metabolischen Zusammenbruch des Nährmediums zu bewirken, wodurch in mindestens einigen der Nährmedien Kohlendioxid gebildet wird,

(c) die Anwesenheit oder Abwesenheit von Kohlendioxid wird in der aus jedem Nährmedium entwickelten gasförmigen Atmosphären durch Messen der Infrarotabsorption der gasförmigen Atmosphäre bestimmt, indem man einen Infrarotstrahl durch die gasförmige Atmosphäre hindurchtreten läßt und die Infrarotabsorption der gasförmigen Atmosphäre bestimmt,

(d) für den bekannten Mikroorganismus wird ein Standard-Kohlendioxid-Profil auf Basis der Verwendung von Nährmedien erstellt, bei denen die Anwesenheit oder Abwesenheit von Kohlendioxid durch Infrarotabsorption bestimmt wird;

(iii) die Stufen (a) bis (d) werden für eine Vielzahl von bekannten Mikroorganismen wiederholt, um eine Reihe von Standard-Kohlendioxid-Profilen für bekannte Mikroorganismen zu erstellen;

(iv) die Stufen (a) bis (d) werden für eine Probe wiederholt, die einen unbekannten Mikroorganismus enthält, um ein unbekanntes Kohlendioxid-Profil zu erstellen; und

(v) das unbekannte Kohlendioxid-Profil wird mit der Reihe von Standard-Kohlendioxid-Profilen in der Absicht verglichen, den unbekannten Mikroorganismus zu identifizieren.

2. Verfahren nach Anspruch 1, bei dem die Infrarotabsorption in Glasbehältern bei etwa 2360 cm$^{-1}$ und bei etwa 2400 cm$^{-1}$ gemessen wird und die Absorption bei etwa 2400 cm$^{-1}$ von der Absorption bei etwa 2360 cm$^{-1}$ abgezogen wird, um so eine korrigierte Messung der auf Kohlendioxid zurückzuführenden Absorption bei etwa 2360 cm$^{-1}$ zu erhalten.

3. Verfahren nach Anspruch 1, bei dem die Infrarotabsorption in Polymethylpenten-Behältern bei etwa 2360 cm$^{-1}$ und bei etwa 2250 cm$^{-1}$

gemessen wird und die Absorption bei etwa 2250 cm$^{-1}$ von der Absorption bei etwa 2360 cm$^{-1}$ abgezogen wird, um so eine korrigierte Messung der auf Kohlendioxid zurückzuführenden Absorption bei etwa 2360 cm$^{-1}$ zu erhalten.

4. Verfahren nach Anspruch 1, bei dem die Infrarot-Extinktion (negativer dekadischer Logarithmus des Durchlaßgrades) bei etwa 670 cm$^{-1}$ gemessen wird.

5. Verfahren nach einem der Ansprüche 1 bis 4, bei dem die Inkubation 1 bis 24 Stunden lang durchgeführt wird.

6. Verfahren nach Anspruch 5, bei dem die Inkubation 2 bis 8 Stunden lang durchgeführt wird.

7. Verfahren nach einem der Ansprüche 1 bis 6, bei dem die Kohlenstoffquelle ausgewählt wird aus einem Kohlenhydrat, einem Kohlenhydrat-Derivat, einem Polyol, Harnstoff, Zitronensäure, einer Fettsäure, einer Aminosäure, einem Peptid mit niedrigem Molekulargewicht oder einer Purin- oder Pyrimidin-Base.

8. Verfahren nach Anspruch 7, bei dem die Kohlenstoffquelle ein Kohlenhydrat ist.

9. Verfahren nach Anspruch 8, bei dem das Kohlenhydrat ausgewählt wird aus Glucose, Lactose, Arabinose, Raffinose, Saccharose, Rhamnose oder Trehalose.

10. Verfahren nach Anspruch 7, bei dem die Kohlenstoffquelle eine Aminosäure ist.

11. Verfahren nach Anspruch 10, bei dem die Aminosäure ausgewählt wird aus Arginin, Ornithin, Lysin, Glycin, Alanin, Tyrosin, Threonin, Histidin oder Leucin.

12. Verfahren nach Anspruch 7, bei dem das Kohlenhydrat-Derivat ein Salicin ist oder das Polyol ausgewählt wird aus Glycerin, Mannit, Inosit, Sorbit, Dulcit oder Adonit.

## Revendications

1. Méthode pour l'identification d'un microorganisme dans un échantillon, cette méthode comprenant:

(i) l'alimentation d'une pluralité de conteneurs dans lesquels des conteneurs individuels sont chacun alimentés avec différents milieux de croissance pour microorganismes, chaque milieu de croissance contenant respectivement une source de carbone qui peut être métabolisable pour produire du dioxyde de carbone;

(ii) la préparation d'un profil standard de dioxyde de carbone pour un microorganismes particulier connu grâce aux étapes suivantes:

(a) inoculation d'une pluralité de conteneurs par un échantillon du microorganismes connu;

(b) l'incubation du conteneur pendant un temps suffisant pour provoquer la dégradation métabolique du milieu de croissance d'où résulte la production de dioxyde de carbone dans au moins quelques-uns des milieux de croissance;

(c) la détection de la présence ou de l'absence de dioxyde de carbone dans l'atmosphère gazeuse créée dans chaque milieu de croissance en mesurant l'absorption infrarouge de l'atmosphère gazeuse en faisant passer un faisceau infrarouge à travers l'atmosphère gazeuse et la détection de l'absorption infrarouge dans l'atmosphère gazeuse;

(d) la préparation d'un profil de dioxyde de carbone standard pour le microorganismes connu basé sur l'utilisation de milieux de croissance dans lesquels la présence ou l'absence de dioxyde de carbone est déterminée par absorption infrarouge;

(iii) la répétition des étapes de (a) jusqu'à (d) pour une pluralité de microorganismes connus pour alimenter une série de profils de dioxyde de carbone standards pour des microorganismes connus;

(iv) la répétition des étapes de (a) jusqu'à (d) pour un échantillon contenant un microorganisme inconnu pour alimenter un profil de dioxyde de carbone inconnu; et

(v) la comparaison du profil de dioxyde de carbone inconnu avec la série des profils de dioxyde de carbone standards dans l'intention d'identifier le microorganisme inconnu.

2. Méthode conforme à la revendication 1 dans laquelle l'absorption infrarouge est mesurée dans des conteneurs en verre à approximativement 2 360 cm$^{-1}$ et à approximativement 2 400 cm$^{-1}$, et l'absorption à approximativement 2 400 cm$^{-1}$ est soustraite de l'absorption à approximativement 2 360 cm$^{-1}$ d'où résulte une mesure correcte de l'absorption à approximativement 2 360 cm$^{-1}$ du dioxyde de carbone.

3. Méthode conforme à la revendication 1 dans laquelle l'absorption infrarouge est mesurée dans des conteneurs en polyméthylpentène à approximativement 2 360 cm$^{-1}$ et à approximativement 2 250 cm$^{-1}$ et l'absorption à approximativement 2 250 cm$^{-1}$ est soustraite de l'absorption à approximativement 2 360 cm$^{-1}$ pour fournir une mesure correcte de l'absorption à approximativement 2 360 cm$^{-1}$ due an dioxyde de carbone.

4. Méthode conforme à la revendication 1 dans laquelle l'absorption infrarouge est mesurée à approximativement 670 cm$^{-1}$.

5. Méthode conforme à l'une quelconque des revendications 1 à 4 dans laquelle l'incubation dure de 1 à 24 heures.

6. Méthode conforme à la revendication 5 dans laquelle l'incubation dure de 2 à 8 heures.

7. Méthode conforme à l'une quelconque des revendications 1 à 6 dans laquelle la source de carbone est sélectivement un hydrate de carbone dérivé, un polyol, de l'urée, de l'acide citrique, un acide gras, un acide aminé, une peptide à faible poids moléculaire, ou une purine ou une base pyrimidine.

8. Méthode conforme à la revendication 7 dans laquelle la source de carbone est un hydrate de carbone.

9. Méthode conforme à la revendication 8 dans laquelle l'hydrate de carbone est choisi parmi le glucose, le lactose, l'arabinose, le raffinose, le sucrose, le rhammose ou le treholose.

10. Méthode conforme à la revendication 7 dans laquelle la source de carbone est un acide aminé.

0 151 855

11. Méthode conforme à la revendication 10 dans laquelle ledit acide aminé est choisi dans le groupe de l'arginine, l'ornithine, la lysine, la glycine, l'alanine, la tyrosine, la threonine, l'histidine, ou la leucine.

12. Méthode conforme à la revendication 7 dans laquelle l'hydrate de carbone dérivé est de la salicin ou le polyol choisi dans le groupe du glycérol, du mannitol, de l'inositol, du sorbitol, du dulcitol ou de l'adonitol.

0 151 855

FIG.1

FIG.2

FIG.3

# 0 151 855

IR SCAN OF A BOROSILICATE TUBING VIAL

FIG.4

IR SCAN OF A SODA-LIME BLOOD CULTURE BOTTLE

FIG.5

0 151 855

CARBON DIOXIDE QUANTIFICATION
IN POLYMETHYLPENTENE BOTTLE

FIG.6

6

CARBON DIOXIDE QUANTIFICATION IN A
BOROSILICATE GLASS TUBING VIAL

FIG.7

E. COLI GROWTH STUDY

FIG. 8

IR SPECTRUM OF HEAD SPACE GAS FROM:
A. STERILE MEDIUM CONTROL
B. STERILE MEDIUM WITH CLOSTRIDIUM
   PERFRINGENS

FIG.9

Horizontal axis for all spectra is in wave numbers from 2200 to 2500 cm$^1$. Vertical axis for all spectra is in absorbance units from 0.8 to 4.0 .

FIG.10

Horizontal axis for all spectra is in wave numbers from 2200 to 2500 cm⁻¹. Vertical axis for all spectra is in absorbance units from 0.8 to 4.0.

FIG.11